# FASCICULE DE BREVET EUROPEEN

(11) **EP 3 465 183 B1**
(45) Date de publication et mention de la délivrance du brevet: **29.11.2023**
(21) Numéro de dépôt: 17726660.8
(22) Date de dépôt: 02.06.2017
(51) Int. Cl.: G01N 23/223, G01N 33/28, G01N 33/30

(54) **PROCÉDÉ DE CALIBRATION D'UN CAPTEUR ET MÉTHODE AUTOMATISÉE DE SUIVI EN LIGNE DE L'ÉVOLUTION D'UN CORPS LIQUIDE**
VERFAHREN ZUR KALIBRIERUNG EINES SENSORS UND AUTOMATISIERTES VERFAHREN ZUR ONLINE-ÜBERWACHUNG DER ÄNDERUNGEN AN EINEM FLÜSSIGKÖRPER
PROCEDURE FOR CALIBRATING A SENSOR AND AUTOMATED METHOD FOR ONLINE MONITORING OF THE CHANGES TO A LIQUID BODY

(30) Priorité: 02.06.2016 FR 1655024
(43) Date de publication de la demande: 10.04.2019
(73) Titulaire: Wika Tech S.a.S., 73370 Le Bourget-du-Lac (FR)
(72) Inventeur: TROADEC, Yann, 38000 Grenoble (FR); JUSTON, Raphaël, 38530 Barraux (FR); CHAUDOREILLE, François, 73100 Gresy Sur Aix (FR)
(74) Mandataire: Lavoix
(86) Numéro de dépôt international: PCT/EP2017/063534
(87) Numéro de publication internationale: WO 2017/207790

(56) Documents cités:
- US-A- 5 982 847
- AKIHO HIROYUKI ET AL: "Speciation and oxidation reaction analysis of selenium in aqueous solution using X-ray absorption spectroscopy for management of trace element in FGD liquor", FUEL, vol. 102, 3 juillet 2012 (2012-07-03), pages 156-161, XP028937452, ISSN: 0016-2361, DOI: 10.1016/J.FUEL.2012.06.076
- Y. BALAJI RAO ET AL: "Determination of uranium in process stream solutions from uranium extraction plant employing energy dispersive X-ray fluorescence spectrometry", JOURNAL OF RADIOANALYTICAL AND NUCLEAR CHEMISTRY, vol. 294, no. 3, 21 octobre 2011 (2011-10-21), pages 371-376, XP055335815, HU ISSN: 0236-5731, DOI: 10.1007/s10967-011-1477-0
- MANNING C E ET AL: "Rutile solubility in albite-H2O and Na2Si3O7-H2O at high temperatures and pressures by in-situ synchrotron radiation micro-XRF", EARTH AND PLANETARY SCIENCE LETTERS, NORTH HOLLAND PUBL., CO, NL, vol. 272, no. 3-4, 15 août 2008 (2008-08-15), pages 730-737, XP025876592, ISSN: 0012-821X, DOI: 10.1016/J.EPSL.2008.06.004 [extrait le 2008-06-12]
- DALIÁNGELIS R. NÚÑEZ-MILLAND ET AL: "Quantification of phosphorus in single cells using synchrotron X-ray fluorescence", JOURNAL OF SYNCHROTRON RADIATION., vol. 17, no. 4, 1 juillet 2010 (2010-07-01), pages 560-566, XP055384398, DK ISSN: 0909-0495, DOI: 10.1107/S0909049510014020
- Rigaku: "Application package for X-ray fluorescence analysis - Simple, fast calibration for quantitative analysis", , 1 janvier 2010 (2010-01-01), XP055384387, Extrait de l'Internet: URL:https://www.rigaku.com/downloads/journ al/RJ26-2/RJ26-2_31-32.pdf [extrait le 2017-06-22]

## Description

L'invention a trait à un procédé de calibration d'un capteur de détermination de la teneur globale, en un élément chimique prédéterminé, d'un corps liquide. L'invention a également trait à une méthode automatisée de suivi en ligne de l'évolution de la teneur globale en un élément chimique prédéterminé, d'un corps liquide qui comprend la mise en oeuvre d'un tel procédé de calibration..

Le domaine de l'invention est celui des instruments de mesure pour des corps liquides et des services associés, notamment dans le domaine de la production d'énergie renouvelable, du raffinage, du transport terrestre, de la chimie et de la pharmacie.

Dans le domaine des énergies renouvelables, il est connu qu'il convient de suivre la situation d'un équipement en analysant un liquide circulant dans cet équipement. Par exemple, il est connu de suivre la composition d'une huile de lubrification d'une éolienne. Une telle analyse permet de détecter des phénomènes d'usure ou de corrosion qui ont tendance à se produire dans la boite de vitesse d'une éolienne. Les opérations de maintenance sont coûteuses et, actuellement, le contrôle du liquide de lubrification des éoliennes est effectué, après prélèvement, en laboratoire. Les éoliennes sont sensibles aux phénomènes d'usure ou de corrosion, leurs difficultés d'accès imposent que ces analyses soient réalisées sur site, notamment pour suivre la teneur globale en fer particulaire de l'huile, cette teneur résultant de phénomènes d'abrasion. La teneur globale en fer de l'huile comprend la teneur en fer présent dans l'huile sous forme particulaire, par exemple en tant qu'oxyde de fer, et sous la forme dissoute, par exemple sous la forme ionique. Ceci impose de former le personnel et de déplacer sur site un matériel élaboré, dont le fonctionnement est relativement difficile à maitriser, même par un personnel formé. En outre, ceci augmente la charge de travail du personnel.

WO-A-2010/046591 prévoit d'utiliser un système embarqué dans lequel l'huile sortant d'un moteur est dirigée vers un composant fonctionnel associé à un système de mesure permettant de déterminer son indice de basicité ou sa teneur en particules métalliques. En pratique, le débit d'huile sortant du moteur est faible et l'écoulement en sortie du moteur est constitué de gouttelettes qui ruissèlent à l'intérieur d'une canalisation, au point qu'il n'est pas certain que le composant fonctionnel soit alimenté avec un débit d'huile suffisant pour que les mesures qu'il effectue soient correctes.

WO-A-03/091550 divulgue une méthode d'analyse d'un corps liquide dans laquelle une mesure, effectuée au moyen d'un capteur XRF sur un échantillon d'un corps liquide à contrôler, est comparée à des mesures effectuées sur des échantillons de référence. Cette démarche est également envisagée dans l'article « Rutile solubility in albite-H20 and Na2Si3O7-H20 at high températures and pressures by in-situ synchrotron radiation micro-XRF » de Manning et al (Earth and Plantary Science Letters - vol.272(2008) - pages 730-737). Cette démarche est prévue pour un fonctionnement en laboratoire et requiert une main d'oeuvre qualifiée.

Il est par ailleurs connu de US-A-5 982 847 d'utiliser un spectromètre comprenant une source de rayons X et un détecteur de rayons X associé à une cellule réalisée dans un matériau non métallique ou à base d'aluminium, dans laquelle circule un lubrifiant et qui est équipé d'une fenêtre transparente aux rayons X. Ce spectromètre, qui fonctionne sur la base de la technologie de fluorescence X, requiert d'être calibré ou étalonné de façon régulière, afin d'éviter les erreurs de mesure.

D'autre part, les équipements de laboratoires, tels que celui connu de US-B-6 233 307 qui pourrait permettre de détecter la teneur en fer dissout d'une huile pour une éolienne, sont difficilement transportables et d'une utilisation complexe, ce qui les rend peu pratiques, même pour un personnel nomade formé pour cela. Ce type de matériel requiert également d'être régulièrement calibré, ce qui est complexe à mettre en oeuvre.

En effet, lors de la calibration des spectromètres connus, on utilise un ou plusieurs échantillons témoins de corps liquide sur lequel ou lesquels doivent être effectuées des mesures, avant que le résultat des mesures soit comparé à des données stockées en mémoire. Une telle approche est envisageable en laboratoire mais ne peut en pratique pas être mise en oeuvre dans une installation embarquée qui n'est pas prévue pour cela.

Des problèmes analogues se posent pour les capteurs de détermination de la teneur d'un corps liquide en un autre élément chimique prédéterminé, notamment en calcium, soufre, vanadium, chrome, molybdène, cuivre, argent, étain, aluminium, nickel, zinc, plomb ou en phosphore
Des problèmes analogues se posent également lorsqu'on souhaite connaître, de façon fiable, la teneur en un élément chimique prédéterminé dans des corps liquides. Par exemple, dans le domaine du raffinage, il peut être opportun de connaître la teneur du fuel lourd traité en un métal donné. C'est pourquoi, la présente invention trouve application avec différents corps liquides susceptibles de comprendre un ou plusieurs éléments chimiques prédéterminés.

C'est à ces inconvénients qu'entend plus particulièrement remédier l'invention en proposant un nouveau procédé de calibration d'un capteur de détermination de la teneur en un élément chimique prédéterminé d'un corps liquide, ce capteur utilisant la technologie de fluorescence X et comprenant une source de rayons X, un détecteur de rayons X et une cellule destinée à contenir un échantillon de corps liquide à analyser, cette cellule incluant elle-même un boîtier qui définit un volume interne de réception de l'échantillon. Conformément à l'invention, ce procédé comprend au moins les étapes suivantes consistant à :
établir, en intégrant des mesures effectuées par le détecteur de rayons X sur une période prédéterminée pour chaque niveau d'énergie qui correspond à une longueur d'onde caractéristique d'un composant chimique, un premier spectre de rayonnement X secondaire comprenant des niveaux d'énergie correspondant aux composants du matériau du boîtier ;
b) stocker (200) dans une mémoire le premier spectre (S1), sous la forme d'un ensemble de données (D1) ;
c) faire fonctionner le capteur, alors que le volume interne du boîtier ne contient pas de corps liquide et établir un deuxième spectre de rayonnement X secondaire
d) modifier le deuxième spectre, en utilisant le premier spectre stocké à l'étape b) comme spectre de base, pour aligner les niveaux d'énergie remarquables du deuxième spectre sur ceux du premier spectre, en repérant quel pic du premier spectre correspond à chaque pic du deuxième spectre et en corrigeant les valeurs des niveaux d'énergie remarquables du deuxième spectre pour les disposer, sur l'axe des abscisses, au même emplacement que les niveaux d'énergie remarquables du premier spectre ;
e) enregistrer le deuxième spectre modifié comme spectre de référence pour le capteur.

Le procédé de calibration conforme à l'invention permet donc de se passer d'échantillons de corps liquide à analyser et de se passer d'introduire de tels échantillons dans une installation qui incorpore le capteur, en utilisant comme spectre de référence un spectre de référence basé sur les composants du boîtier de la cellule de mesure de ce capteur. L'invention prend donc le contre-pied des techniques classiques de calibration qui consistent à utiliser des échantillons représentatifs du produit à analyser. En effet, le procédé de l'invention base la calibration sur un matériau différent de celui qui est analysé, à savoir le matériau du boîtier. Le procédé de calibration peut donc être mis en oeuvre de façon automatique, sans avoir à mettre en place un échantillon du corps liquide dans la cellule du capteur, ou de façon manuelle par une personne sans compétence spécifique dans le domaine chimique, en particulier un agent nomade chargé de l'entretien des éoliennes ou un technicien de raffinerie.

Selon des aspects avantageux mais non obligatoires de l'invention, un tel procédé peut incorporer une ou plusieurs des caractéristiques suivantes, prises dans toute combinaison techniquement admissible :
- Lors de l'étape b), le volume interne du boîtier contient exclusivement un gaz ou un mélange gazeux, inerte aux rayons X, notamment de l'air.
- Le boîtier est métallique et les niveaux d'énergie remarquables du premier spectre de référence comprennent des niveaux d'énergie de métaux composant le coffret.
- L'élément chimique prédéterminé dont on souhaite déterminer la teneur est le fer et les métaux dont les niveaux d'énergie constituent le premier spectre ne comprennent pas le fer.
- Le premier spectre comprend également au moins un niveau d'énergie correspondant au matériau d'une anode de la source de rayons X.

Selon un autre aspect, l'invention concerne une méthode automatisée de suivi en ligne de l'évolution de la teneur en un élément chimique prédéterminé d'un corps liquide circulant dans un équipement au moyen d'une installation comprenant un capteur de la teneur en élément chimique prédéterminé de ce corps liquide, ce capteur utilisant la technologie de fluorescence X et comprenant une source de rayons X, un détecteur de rayons X et une cellule destinée à contenir un échantillon de corps liquide à analyser et incluant un boîtier définissant un volume interne de réception de cet échantillon. Conformément à l'invention, cette méthode comprend la mise en oeuvre préalable d'un procédé de calibration du capteur, tel qu'envisagé ci-dessus, ainsi qu'au moins une série d'étapes consistant à déterminer, grâce au capteur préalablement calibré, la teneur en élément chimique prédéterminé d'un échantillon de corps liquide présent ou circulant dans le volume interne du boîtier, cette série d'étapes comprenant au moins les étapes suivantes consistant à
f) démarrer le capteur ;
g) acquérir un spectre avec un fonctionnement analogue à celui mis en oeuvre à l'étape c) ;
h) comparer le spectre acquis à l'étape g) au spectre de référence enregistré à l'étape e) ; et
i) comparer un nombre de coups, correspondant à un niveau d'énergie représentatif de l'élément chimique prédéterminé, au nombre de pics détectés pour des échantillons de référence.

Selon d'autres aspects avantageux mais non obligatoires de l'invention, cette méthode peut incorporer une ou plusieurs des caractéristiques suivantes, prises selon toute combinaison techniquement admissible :
- L'élément chimique prédéterminé est le fer, ou un autre élément chimique, et la série d'étapes comprend la détection de pics pour un niveau d'énergie égal à environ 6,4 keV et/ou 7,06 keV dans un spectre de rayonnement X secondaire émis par le corps liquide pour le fer, ou au raies spécifiques pour l'autre élément chimique prédéterminé.
- Lors de la série d'étapes, il est tenu compte d'un éventuel effet de matrice au sein de l'échantillon en appliquant une correction sur les niveaux d'énergie détectés par le capteur, sur la base d'un modèle prédéterminé d'interaction entre les composants de l'échantillon et/ou les composants du boîtier.
- Dans le cas où il est tenu compte de l'effet de la matrice, lors de la série d'étapes de correction des effets de matrices, il est, en outre, tenu compte de la concentration en calcium de l'échantillon en appliquant une correction sur les niveaux d'énergie détectés par le capteur, sur la base d'un modèle prédéterminé.
- La détermination de la teneur en élément chimique prédéterminée de l'échantillon est effectuée de façon dynamique ou statique.

L'invention sera mieux comprise et d'autres avantages de celle-ci apparaitront plus clairement à la lumière de la description qui va suivre de deux modes de réalisation d'une installation conforme à son principe, donnée uniquement à titre d'exemple et faite en référence aux dessins annexés dans lesquels :
- la figure 1 est une représentation schématique de principe d'une installation incorporant un capteur,
- la figure 2 est une vue à plus grande échelle du détail II à la figure 1, montrant un capteur de détermination de la teneur en un élément chimique prédéterminé, notamment en fer, utilisé dans l'installation de la figure 1,
- la figure 3 est une vue à plus grande échelle du détail III à la figure 2,
- la figure 4 est une vue en perspective du capteur représenté aux figures 2 et 3,
- la figure 5 est une vue en perspective, selon un autre angle, du capteur des figures 2 à 4,
- la figure 6 est un schéma bloc d'un procédé de calibration conforme à l'invention,
- la figure 7 est un schéma bloc d'une partie d'une méthode de suivi en ligne de l'évolution de la teneur en fer d'un corps liquide, également conforme à l'invention,
- la figure 8 est une représentation schématique à plus petite échelle de la partie fluidique de l'installation de la figure 1 dans une première configuration d'utilisation, et
- les figures 9 à 11 sont des vues analogues à la figure 8 lorsque l'installation est dans une deuxième, une troisième et une quatrième configurations d'utilisation.

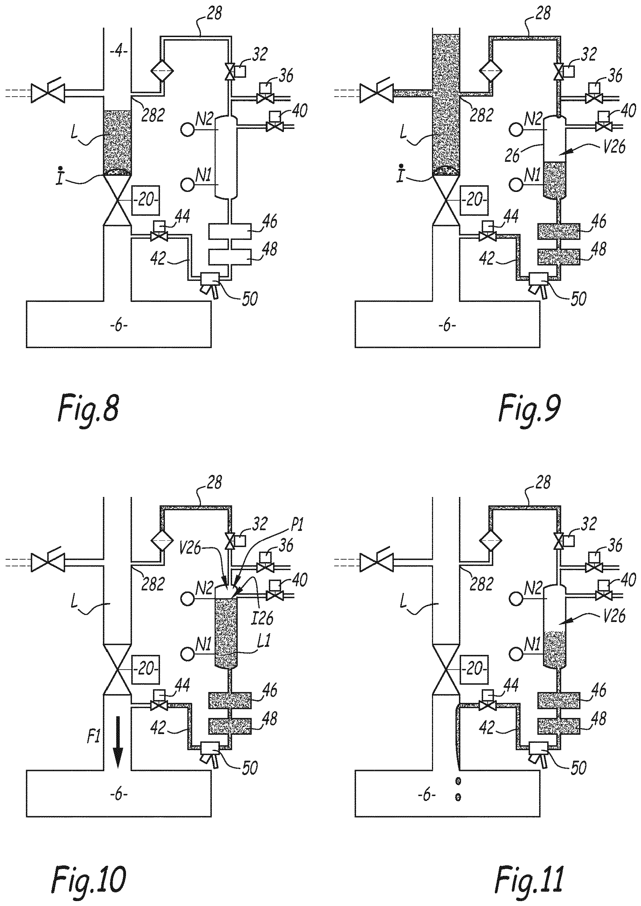

Sur les figures 8 à 11, le corps liquide présent ou circulant dans une partie de l'installation est représenté en grisé.

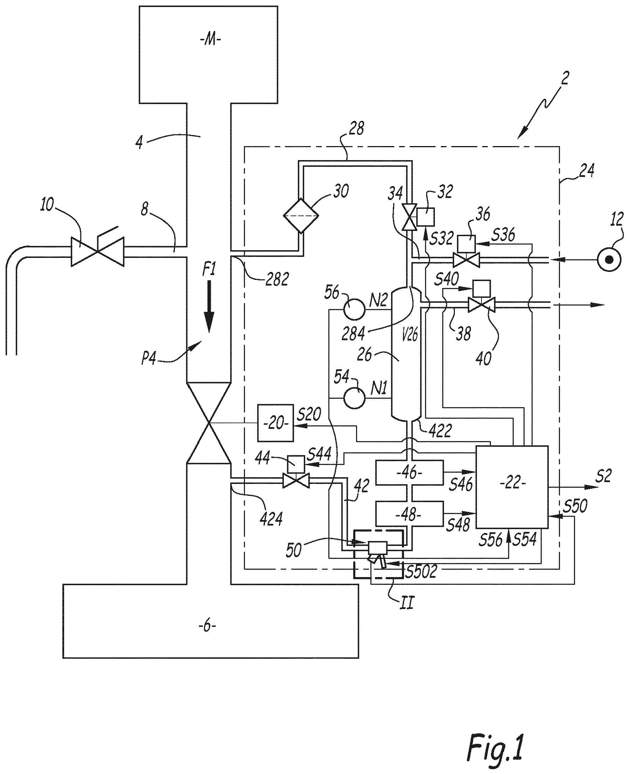

L'installation 2 représentée aux figures 1 à 5 et 8 à 11 permet de suivre automatiquement l'évolution de la teneur globale en fer d'un corps liquide formé par un lubrifiant utilisé dans un équipement, en l'espèce une machine M de type éolienne. Une conduite 4 relie l'équipement M à un bac 6 de récupération de lubrifiant. En pratique, l'huile de lubrification de l'équipement M s'écoule dans la conduite 4 avec une pression P4 comprise entre 1,1 et 6 bars absolus. Le débit d'huile dans la conduite 4 peut être faible, au point que l'huile ruisselle sur la paroi interne de cette conduite.

Un piquage 8 est prévu sur la conduite 4 et équipé d'une vanne 10 commandée manuellement, ce qui permet de prélever une quantité d'huile sortant de l'équipement M afin de procéder à des analyses physico-chimiques, selon une approche connue en soi.

L'installation 2 comprend une vanne d'arrêt 20 montée sur la conduite 4 et qui permet d'interrompre, de façon sélective, l'écoulement d'huile dans la conduite 4, en direction du bac 6. La vanne d'arrêt 20 est commandée par une unité électronique 22 au moyen d'un signal électrique S20.

Comme visible uniquement à la figure 1, l'installation 2 comprend un coffret 24, représenté par sa trace en trait d'axe et à l'intérieur duquel sont disposés les éléments constitutifs de l'installation 2, à l'exception de la partie de la vanne d'arrêt 20 qui est intégrée à la conduite 4.

L'installation 2 comprend également un réservoir tampon 26 qui est disposé dans le coffret 24 et qui est relié à la conduite 4 au moyen d'une première ligne de dérivation 28.

On note 282 l'embouchure de la ligne 28. Cette embouchure est disposée en amont de la vanne 20 sur la conduite 4. La première ligne de dérivation 28 est équipée, en aval de son embouchure 282 vers son débouché 284 dans le réservoir tampon 26, d'un filtre 30, d'une vanne d'arrêt 32 et d'un piquage 34. Le filtre 30 sert à empêcher des impuretés de taille trop importante de s'écouler dans la première ligne de dérivation 28. La vanne d'arrêt 32 permet, au choix, de rendre passante ou de fermer la première ligne de dérivation 28. La vanne 32 est commandée par l'unité électronique 22, au moyen d'un signal électrique S32. Le piquage 34 est relié, à travers une vanne commandée 36, à une source 12 d'air sous pression qui ne fait pas partie de l'installation 2 mais appartient aux équipements standards d'éolienne.

En pratique, la source 12 d'air sous pression peut être un compresseur installé dans l'éolienne et qui alimente un réseau d'air comprimé qui sert également à d'autres équipements que l'installation 2. En variante, la source 12 peut être une pompe dédiée à l'installation 2.

L'installation 2 comprend également un piquage 38 raccordé au réservoir 26, sur lequel est montée une vanne d'arrêt 40 et qui permet de mettre en communication le volume intérieur V26 du réservoir 26 avec l'atmosphère ambiante.

Dans ce mode de réalisation, les piquages 34 et 38 sont indépendants. En variante, ils peuvent être remplacés par un unique piquage, raccordé à la première ligne 28 ou directement au réservoir 26, sur lequel les vannes 36 et 40 sont montées en parallèle, en étant respectivement reliées à la source 12 d'air sous pression et à l'atmosphère ambiante. Dans ce cas, il est possible de combiner les vannes 36 et 40 sous la forme d'une unique vanne trois voies.

Les vannes 36 et 40 sont commandées par l'unité électronique 22 au moyen de signaux électriques respectifs S36 et S40.

L'installation 2 comprend également une deuxième ligne 42 d'évacuation du lubrifiant, du volume intérieur V26 du réservoir 26 vers le bac de récupération 6. La deuxième ligne d'évacuation 42 est donc disposée en aval de la première ligne de dérivation 28 et du réservoir 26, sur le trajet d'écoulement du lubrifiant. Dans l'exemple, la deuxième ligne 42 s'étend du réservoir 26 vers la conduite 4. Son embouchure 422 est située en partie basse du réservoir 26, alors que son débouché 424 est disposé sur la conduite 4, en aval de la vanne d'arrêt 20, comme représenté sur les figures, ce qui permet de réduire le temps d'un cycle d'analyse car la vanne d'arrêt 20 peut être fermée pour créer une colonne d'huile dans la conduite 4, alors que des étapes de mesure ont lieu. En variante, le débouché 424 de la deuxième ligne 42 est disposé en amont de la vanne d'arrêt 20, ce qui permet d'exécuter simultanément des étapes de vidange et de décolmatage du filtre 30 et, éventuellement, de réduire le coût de l'installation 2.

La deuxième ligne 42 est équipée d'une vanne d'arrêt 44 qui est commandée par l'unité électronique 22 au moyen d'un signal électrique S44.

Trois capteurs 46, 48 et 50 sont disposés sur la ligne 42, en amont de la vanne 44.

Le capteur 46 permet de mesurer la densité D, la viscosité V, l'humidité H et la température T d'un corps liquide présent ou s'écoulant dans la deuxième ligne 42, tel que l'huile de lubrification sortant de l'équipement M. Ce capteur peut être du type de celui commercialisé par la société AVENISENSE sous la dénomination Cactus. En variante, le capteur 46 peut être d'un autre type ou ne permettre de mesurer qu'un seul ou certains des paramètres mentionnés ci-dessus.

Le capteur 48 est un capteur d'indice de basicité ou BN, parfois dénommé indice d'alcalinité. Il peut s'agir d'un capteur fonctionnant avec la technologie infrarouge, dans le moyen infrarouge, ou de tout autre capteur adapté à la détermination du BN d'un lubrifiant.

Le capteur 50 est un capteur de détermination de la teneur globale en fer, c'est-à-dire la teneur en fer dissout et/ou particulaire, d'un échantillon de corps liquide, ici de lubrifiant, sortant du réservoir 26, au moyen de la technologie de fluorescence X.

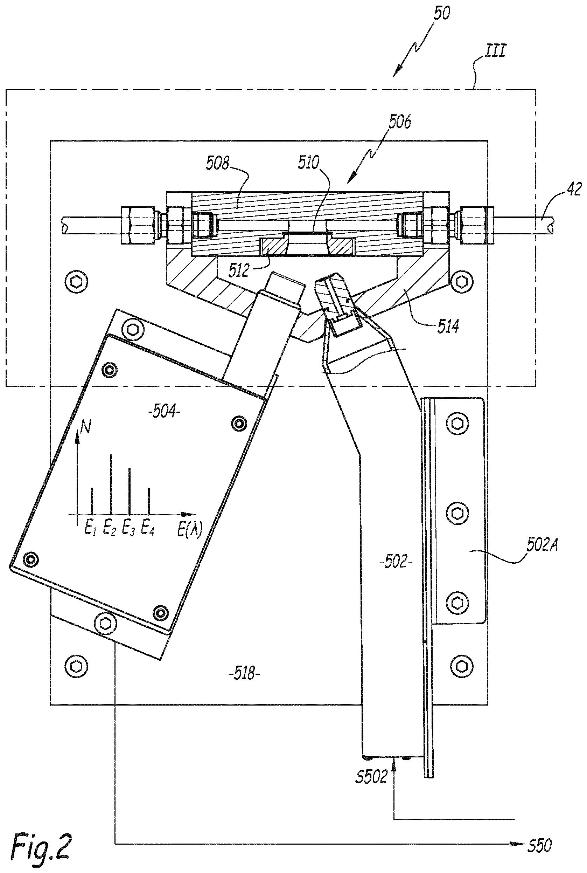

Comme cela ressort plus particulièrement des figures 2 à 5, le capteur 50 comprend une source 502 de rayon X, un détecteur 504 de rayon X et une cellule 506 qui est montée en série sur la deuxième ligne 42. Pour ce faire, la cellule 506 est pourvue d'un élément de raccord amont 506A qui coopère avec l'élément de raccord complémentaire 42A prévu sur la ligne 42, ainsi que d'un élément de raccord aval 506B qui coopère avec un élément de raccord complémentaire 42B prévu sur la ligne 42.

La source 502 comprend une cathode et une anode entre lesquelles circulent des électrons, sous l'effet d'une différence de potentiel de l'ordre de 50 kV, un courant de l'ordre de 500 mA circulant dans la cathode. L'anode est réalisée en métal, par exemple en or, en tungstène, en argent ou en rhodium. La puissance requise pour la source 502 est relativement modeste, notamment comprise entre 4 et 10 W. Un collimateur 502C est utilisé en sortie de la source 502 pour concentrer le faisceau d'électrons centré sur un axe de visée A502 de la source 502.

Les rayons émis par la source X sont dans la gamme des rayons X, avec une longueur d'onde comprise entre 0,01 et 10 nm, c'est-à-dire une fréquence comprise entre environ 3 × 10¹⁹ et 3 × 10¹⁶ Hz.

Le détecteur 504 est de type SDD (de l'Anglais Silicon Drift Detector) qui comprend une électrode unique en face avant, laquelle collecte les électrons générés par l'interaction des rayons X dans la jonction PN d'une photo diode. Ce type de détecteur présente l'avantage d'une faible capacitance due à la faible surface de son anode. Ce type de détecteur permet d'obtenir un taux de comptage élevé, une bonne résolution et un refroidissement efficace par effet Peltier. En variante, le détecteur 504 est de type SI-PIN, avec une photo diode en silicium, qui présente une zone intrinsèque intercalée entre ses deux zones respectivement dopées positivement et négativement.

Le détecteur 504 est capable de compter les « coups » d'émission à chaque niveau d'énergie sur une période donnée, ce qui permet d'établir un spectre de niveaux d'énergie. On note A504 l'axe de visée du détecteur 504, qui correspond à la direction principale des rayons X détectés par ce détecteur.

La cellule 506 comprend un corps 508 réalisé par usinage ou moulage d'un bloc de métal. Ce corps 508 est, de préférence, réalisé en aluminium ou en alliage à base d'aluminium, par exemple en Zicral (7075) qui est un alliage d'aluminium avec le zinc comme élément d'alliage principal. En variante, le boîtier 508 peut être réalisé dans un autre alliage à base d'aluminium. Au sens de la présente description, un alliage à base d'aluminium est un alliage qui comprend au moins 50% en poids d'aluminium. L'utilisation d'un alliage à base d'aluminium permet au boîtier 508 de résister à la température, à la pression et à la composition chimique du lubrifiant s'écoulant dans la ligne 42. L'absence ou la faible proportion de fer contenue dans cet alliage évite que la mesure par fluorescence X de la teneur globale en fer du lubrifiant soit perturbée par la présence de fer en quantité significative.

Toutefois, en variante, il est possible de prévoir que le boîtier 508 soit réalisé en acier inoxydable.

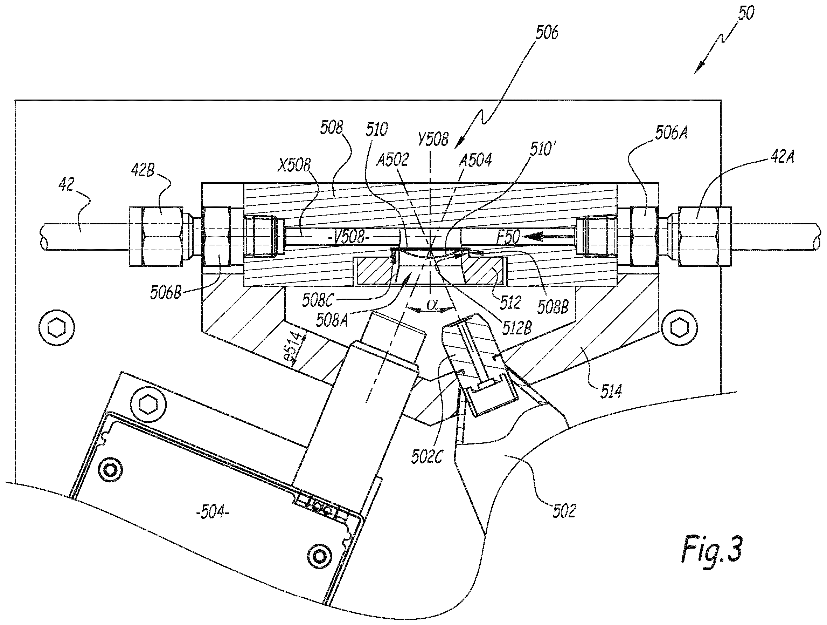

Le boîtier 508 définit un volume V508 de circulation du lubrifiant entre les éléments de raccord 506A et 506B, dans le sens de la flèche F50 à la figure 3. Ce volume V508 est de forme tubulaire à section circulaire ou rectangulaire, au choix du concepteur du boîtier 508. On note X508 un axe longitudinal du volume V508. En pratique, le volume V508 a une contenance comprise entre 1 et 5 ml. Ainsi, l'utilisation du capteur 50 dans le cadre de la présente invention requiert de prélever une quantité d'huile relativement minime dans la conduite 4, ce qui limite les perturbations dans le fonctionnement de la machine M.

Le boîtier 508 est pourvu d'un perçage 508A, qui est centré sur un axe Y508 perpendiculaire à l'axe X508 et qui débouche dans le volume V508. Le perçage 508A est à section circulaire centrée sur l'axe Y508 et pourvu d'un taraudage 508B.

Le perçage 508A est obturé par une paroi 510 en forme de disque, que l'on peut également qualifier de « membrane » et qui est maintenue en appui contre le fond d'un lamage 508C du perçage 508A au moyen d'une bague 512 pourvue d'un filetage externe 512B complémentaire du taraudage 508B et qui coopère avec celui-ci. La paroi ou membrane 510 est montée sur le boîtier 508 en la plaquant contre le lamage 508C, puis en vissant la bague 512 dans le perçage 508A.

La bague 512 est réalisée dans le même métal ou le même alliage que le corps 508.

La paroi 510 constitue une fenêtre de visée pour la source 502 et pour le détecteur 504 qui permet aux rayons X de transiter de la source 502 vers un échantillon de lubrifiant contenu dans le volume V508 et du volume 508 vers le détecteur 504.

La paroi 510 est réalisée en poly(téréphtalate d'éthylène) ou PET, ce qui lui confère des propriétés mécaniques satisfaisantes, alors qu'elle peut avoir une épaisseur faible, inférieure ou égale à 500 µm, de préférence de l'ordre de 250 ou 125 µm, au point qu'elle ne perturbe pas les rayons X qui proviennent de la source 502 ou qui partent vers le détecteur 504.

Selon un mode de réalisation, l'épaisseur de la paroi 510, qui est mesurée perpendiculairement à l'axe Y508, peut être choisie inférieure à 200 µm, de préférence 150 µm, de préférence encore de l'ordre de 125 µm. Selon un autre mode de réalisation, l'épaisseur de la paroi 510 peut être choisie inférieure ou égale à 500 µm, de préférence de l'ordre de 250 µm. Cette épaisseur est supérieure ou égale à 75 µm, ce qui confère une bonne robustesse à la cellule 506.

Selon un aspect optionnel et avantageux représenté en pointillés uniquement à la figure 3 avec la référence 510', la membrane 510 peut présenter une forme bombée, avec sa concavité tournée vers le volume V508. Dans ce cas, la membrane est, par exemple recuite à une température comprise entre 40 et 70 °C, de préférence égale à environ 60°C, pendant une période comprise entre 30 et 60 mn, de préférence égale à environ 60 mn. Ceci permet de s'approcher de la transition vitreuse du PET constituant la membrane 510 et de conférer une rigidité satisfaisante à cette membrane, avec la forme bombée précitée.

Cette forme bombée et/ou cette rigidité permettent à la membrane de résister à des variations de pression au sein du volume V508. Ces variations de pressions résultent, notamment, des pertes de charges dans les lignes et réservoir d'alimentation du capteur 50 et des cycles de remplissage/vidange de ce volume. Ainsi, la membrane 510 peut résister à une pression de0 à 20 bar en absolu, sans risque d'affaissement de la membrane 510 vers l'intérieur du boîtier 508.

La source 502 et le détecteur 504 sont montés sur un capot 514 qui détermine leur position par rapport à la cellule 506, plus particulièrement par rapport au boîtier 508 et à la paroi 510. Ce capot 504 entoure le boîtier 508 du côté du perçage 508A, de sorte qu'il isole la fenêtre formée par la paroi 510 de l'extérieur du capteur 50. On note e514 l'épaisseur du capot 514. Le matériau de ce capot 514 et son épaisseur e514 sont choisis de telle sorte qu'ils constituent un blindage efficace contre les rayons X qui circulent entre la source 502, le détecteur 504 et la cellule 506. Cette cellule permet le libre passage de l'huile et permet d'effectuer des analyses d'huile statiques ou dynamiques. En pratique, le capot 514 peut être réalisé en inox, par exemple de type 316, et l'épaisseur e514 est choisie supérieure à 5 mm, de préférence supérieure à 8 mm, de préférence encore de l'ordre de 10 mm.

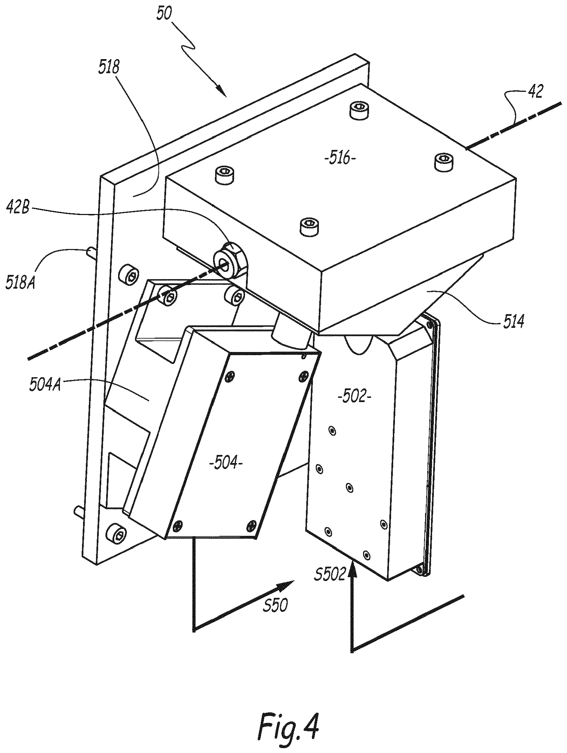

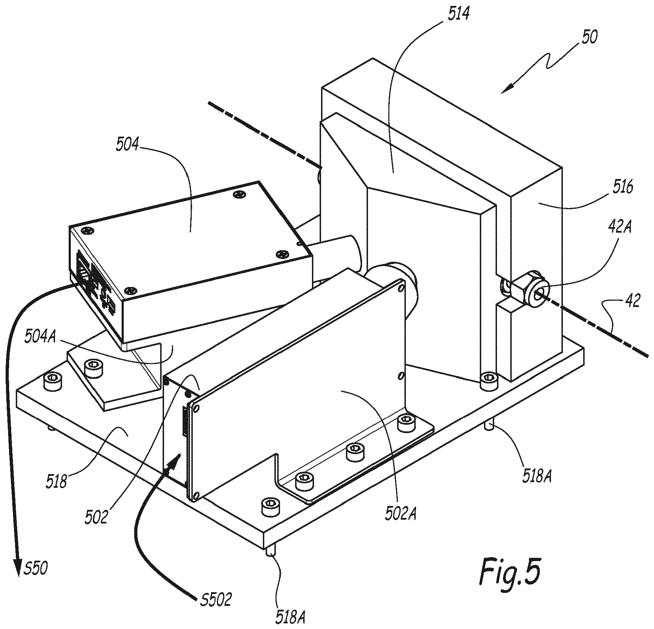

D'autre part, un blindage complémentaire 516 est monté autour du boîtier 508, du côté de ce boîtier opposé au capot 514. Pour la clarté du dessin, ce blindage 516 est représenté uniquement aux figures 4 et 5.

Les parties 502, 504, 506, 514 et 516 du capteur 50 sont fixées sur un support 518 constitué par une plaque qui peut être immobilisée dans le coffret 24 au moyen de vis 518A. Une équerre 502A et une entretoise 54A sont utilisées pour fixer respectivement la source 502 et le détecteur 504 sur le support 518.

Le fait que les parties 502, 504 et 506 sont montées sur le support 518 confère un caractère industriel au capteur 50 dans la mesure où celui-ci peut être manipulé de façon unitaire, en vue de son intégration à l'installation 2 ou lors d'opérations de maintenance. Ceci est beaucoup plus pratique que le cas où la source 502 serait un sous-ensemble complexe, volumineux et onéreux, du type synchrotron qui est un matériel limité à une utilisation en laboratoire.

En pratique, le capteur 50 est inscrit dans un cube imaginaire dont l'arête a une longueur égale à 500 mm, ce qui facilite sa mise en place au sein du coffret 24.

Ainsi assemblé, le capteur 50 constitue un sous-ensemble facile à manipuler, aisément identifiable, et qui peut faire l'objet d'une opération d'échange standard, en dévissant les vis 518A et en séparant les éléments de raccord 506A et 42A, d'une part, 506B et 42B, d'autre part.

La source 502 est commandée par l'unité électronique 22 au moyen d'un signal S502 et le détecteur 504 délivre à l'unité électronique 22 un signal de sortie S50 du capteur 50.

En pratique, les axes X508, Y508, A502 et A504 sont coplanaires.

On note α un angle mesuré entre les axes A502 et A504 à l'extérieur du boîtier 508. Cet angle α a une valeur comprise entre 20 et 25°, de préférence de l'ordre de 22°.

En fonctionnement, la technologie de fluorescence X du capteur 50 est utilisée pour déterminer la teneur globale en fer, c'est-à-dire la teneur en fer dissout et/ou particulaire, d'une quantité d'huile transitant dans le volume V508, en sortie du réservoir tampon 26. La mesure de la teneur en fer est effectuée par le capteur 50 lorsque le lubrifiant s'écoule dans la deuxième ligne 42, c'est-à-dire lorsque la vanne 44 est ouverte. On parle alors de mesure en dynamique. En variante, cette mesure peut être effectuée lorsque le lubrifiant est statique dans le volume V508, c'est-à-dire lorsque la vanne 44 est fermée. On parle alors de mesure en statique.

Que le lubrifiant s'écoule ou soit bloqué dans le corps 508, la mise en oeuvre du capteur 50 implique l'émission par la source 502 d'un faisceau d'électrons, dans la gamme des rayons X, qui traverse la paroi 510 et excite les atomes d'un liquide qui se trouve dans le volume V508, en l'espèce un lubrifiant. Le niveau d'énergie des rayons X issus de la source 502 est compris entre 0 et 30 kilo électron volts (keV). Pour exciter un atome de fer et induire le changement de couche d'un électron dans cet atome, il est nécessaire que le rayonnement X entrant dans le volume V508 ait un niveau d'énergie supérieur au niveau d'énergie caractéristique du fer, soit 6,4 keV.

Afin de déterminer la teneur en fer dissout ou particulaire d'un lubrifiant présent dans le volume V508, le détecteur 504 est réglé pour tenir compte des rayonnements induits avec un niveau d'énergie proche des niveaux d'énergie caractéristiques du fer, soit égal à environ 6,4 keV et/ou 7,06 keV, c'est-à-dire égal à 6,4 keV et/ou 7,06 keV à 5% près.

En pratique, le détecteur 504 est capable de compter, pour chaque niveau d'énergie, le nombre de « coups » correspondants à l'émission d'un photon par changement de couche d'un électron au niveau d'un atome. En intégrant les mesures effectuées par le détecteur 504 sur une période prédéterminée, par exemple égale à trente secondes ou une minute, il est donc possible d'établir un spectre du nombre N de coups détectés sur cette période pour chaque niveau d'énergie E qui correspond à une longueur d'onde λ caractéristique d'un composant chimique. Un tel spectre S est représenté de façon schématique sur le détecteur 504 à la figure 2.

Pour que la mesure effectuée grâce au capteur 50 soit fiable, il faut que le spectre S soit effectivement représentatif en ce qui concerne les niveaux d'énergie E₁, E₂, E₃, E₄ etc... pour lesquels un pic est détecté, c'est-à-dire, en pratique, en ce qui concerne la position des barres verticales du spectre S le long de l'axe des abscisses E qui est l'axe des niveaux d'énergie.

Or, l'intensité des pics d'énergie peut être affectée par la transparence de la paroi 510 ainsi que par l'usure des composants électroniques appartenant aux appareils 502 et 504 ou par la température ambiante. En outre, les paramètres du rayonnement issus de la source 502 peuvent évoluer au cours du temps. On ne peut donc pas se satisfaire d'un unique calibrage du capteur 50 lors de sa fabrication, c'est-à-dire au début de sa durée de vie.

La calibration du capteur 50 a lieu en mettant en oeuvre un procédé de calibration dans lequel on n'utilise pas d'échantillon de lubrifiant à analyser.

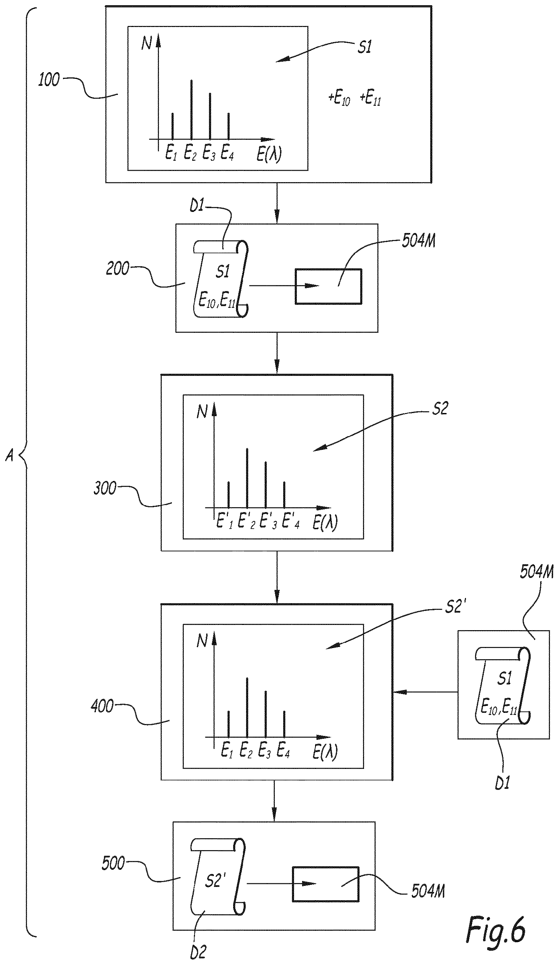

Selon ce procédé, on établit, lors d'une première étape 100 qui peut avoir lieu en laboratoire, un premier spectre S1 de rayonnement X secondaire comprenant des pics de détection pour des niveaux d'énergie correspondant aux composants du matériau du boîtier 508. A la figure 6, quatre niveaux d'énergie E₁, E₂, E₃ et E₄ sont représentés. En pratique, en fonction de la composition du matériau constitutif du boîtier 508, ce nombre peut être supérieur.

De préférence, dans la mesure où le matériau du boîtier 508 est un alliage à base d'aluminium, les niveaux d'énergie E₁, E₂, E₃, E₄, etc. correspondent à des métaux différents du fer.

Après que le premier spectre S1 a été établi lors de l'étape 100, celui-ci peut être stocké, sous la forme d'un ensemble de données D1 et lors d'une étape 200, dans une mémoire 504M du détecteur 504. En variante, le premier ensemble de données D1 correspondant au premier spectre S1 peut être stocké dans une mémoire non représentée de l'unité électronique 22 de l'installation 2.

Les étapes 100 et 200 sont réalisées une seule fois pour chaque capteur 50 lors de la fabrication du capteur 50 si l'ensemble de données est stocké dans la mémoire 504M, ou lors de la fabrication de l'installation 2 s'il est stocké dans la mémoire de l'unité 22.

Dans la mesure où le matériau constitutif du boîtier 508 est déterminé pour une série de capteurs 50, l'étape 100 peut être réalisée une seule fois pour tous ces capteurs puisque le spectre S1 est commun à ces capteurs.

Lors d'une étape subséquente 300 du procédé de calibration, qui peut avoir lieu autant de fois que nécessaire au cours de la durée de vie du capteur 50, on fait fonctionner le capteur 50, alors que le volume V508 est vide de lubrifiant. Ce volume V508 peut être rempli d'un gaz ou d'un mélange gazeux inerte aux rayons X, par exemple de l'air.

Lors de cette étape 300, le capteur 504 établit un deuxième spectre S2 de rayonnement X secondaire.

Si le capteur 50 est parfaitement calibré, le deuxième spectre S2 établi à l'étape 200 et le premier spectre S1 établi à l'étape 100 sont identiques, puisqu'ils concernent le même matériau, à savoir celui du boîtier 508.

Tel n'est pas forcément le cas, pour les raisons envisagées ci-dessus.

Le procédé de calibration de la figure 6 comprend donc une étape supplémentaire 400 au cours de laquelle le deuxième spectre S2 est aligné sur le premier spectre S1 en créant un deuxième spectre modifié S2' qui constitue une version corrigée ou améliorée du deuxième spectre S2, en tenant compte du premier spectre de référence S1. Si les deux spectres S1 et S2 sont identiques, l'étape 400 a lieu mais son effet est nul sur le spectre S2.

En effet, les niveaux d'énergie remarquables E'₁, E'₂, E'₃, E'₄, ... pour lesquels des pics sont détecté dans le spectre S2 peuvent ne pas être rigoureusement égaux aux niveaux d'énergie remarquables E₁, E₂, E₃, E₄, ... correspondant aux composants chimiques de l'alliage constitutif du boîtier 508. Or, en comparant les hauteurs des pics du deuxième spectre S2 à celles des pics du premier spectre S1, il est possible de repérer à quel pic du spectre S1 correspond chaque pic du spectre S2 et de corriger les valeurs des niveaux d'énergie E'₁, E'₂, E'₃, E'₄, ... correspondant aux pics pour les disposer, sur l'axe des abscisses E(λ) du spectre, au même emplacement que les niveaux d'énergie remarquables E₁, E₂, E₃, E₄ du premier spectre S1.

Pour ce faire, au cours de l'étape 400, l'unité 22 ou une unité logique intégrée au détecteur 504 accède à l'ensemble de données D1 dans la mémoire 504M ou dans une mémoire équivalente de l'unité 22 afin de réaliser la comparaison des spectres S1 et S2.

Lors d'une étape suivante 500 du procédé de calibration, le deuxième spectre corrigé S2' est entré dans la mémoire 504M du détecteur 504, sous la forme d'un deuxième ensemble de données D2, pour constituer un spectre de référence pour le capteur 50, spectre de référence auquel sont comparés les pics détectés par le détecteur 504 lors de l'utilisation ultérieure du capteur 50, ainsi qu'il ressort des explications qui suivent.

En variante, l'ensemble de données D2 peut être stocké dans une mémoire de l'unité 22.

Selon un aspect avantageux de l'invention, le premier spectre S1 peut être établi en tenant compte du fait que la nature du matériau constitutif de l'anode de la source de rayons X 502 influe sur les niveaux d'énergie qui correspondent aux pics les plus marqués dans le deuxième spectre collecté S2. En fait, deux niveaux d'énergie caractéristiques E₁₀ et E₁₁ correspondent généralement à des pics particuliers dans le deuxième spectre S2, ces niveaux d'énergie étant connus pour les matériaux les plus couramment utilisés pour constituer l'anode d'une source de rayons X. Ainsi, pour une anode en or, des pics sont constatés pour des niveaux d'énergie de 9,71 et 11,44 keV. Pour une anode en argent, des pics sont constatés pour des niveaux d'énergie de 2,98 keV et 3,15 keV. Pour une anode en tungstène, des pics sont constatés pour des niveaux d'énergie de 8,40 et 9,60 keV. Pour une anode en rhodium, des pics sont constatés pour des niveaux d'énergie de 2,70 keV et 2,83 keV.

Comme le rayonnement X secondaire est issu du rayonnement X primaire, ce rayonnement secondaire, tel que détecté par le détecteur 504 du capteur 50, comprend normalement des pics pour les niveaux d'énergie E₁₀ et E₁₁ du matériau de l'anode.

Le matériau constitutif de l'anode de la source de rayons X502 étant connu, les niveaux d'énergie E₁₀ et E₁₁ sont connus et peuvent être intégrés à l'ensemble de données D1 du premier spectre S1 pour être utilisés pour aligner ou « recaler » le deuxième spectre S2 sur le premier spectre S1 lors de l'étape 400. Dans ce cas, le premier spectre S1, tel qu'il est stocké dans la mémoire 504M pour être utilisé à l'étape 400, comprend également les niveaux d'énergie E₁₀ et E₁₁.

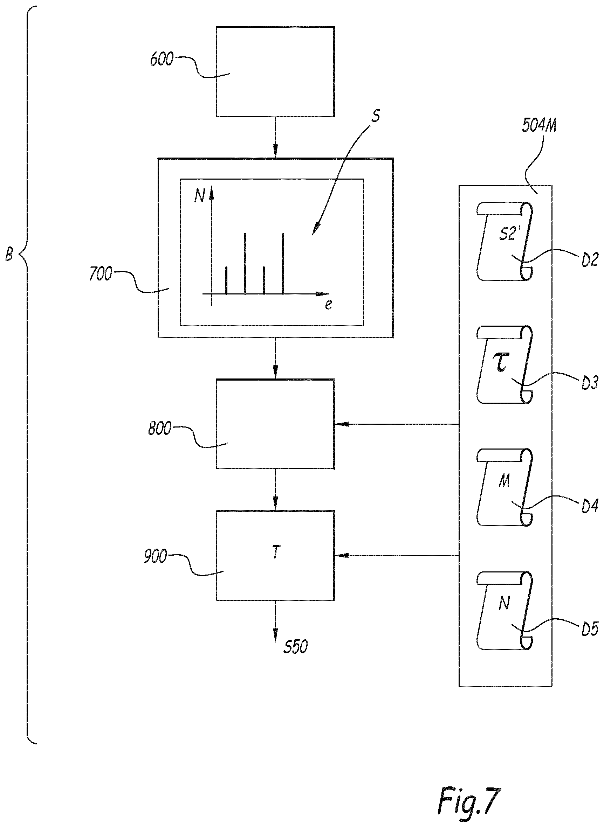

Comme visible à la figure 7, lorsqu'on utilise le capteur 50 pour suivre en ligne l'évolution de la teneur en fer d'un lubrifiant circulant dans l'équipement M, on démarre le capteur lors d'une étape 600 et le détecteur 504 acquiert, lors d'une étape 700, un spectre S, avec un fonctionnement analogue à celui mis en oeuvre lors de l'étape 300, le volume V508 étant alors rempli de lubrifiant à analyser, en cours d'écoulement.

Lors d'une étape ultérieure 800, le spectre acquis S est comparé au spectre de référence S2' pour repérer le nombre de pics correspondant à un niveau d'énergie égal à environ 6,4 keV et/ou 7,06 keV, c'est-à-dire égal à 6,4 keV et/ou 7,06 keV, plus ou moins 5%.

Le nombre de coups correspondant au niveau d'énergie de 6,4 keV représentatif du fer étant connu, il est possible de le comparer, lors d'une étape 900, au nombre de pics détectés pour des échantillons de référence, lors de manipulations réalisées en laboratoire où chaque teneur en fer dissout et en fer particulaire a été associée à un nombre de coups, c'est-à-dire à une hauteur de pic, pour la valeur de 6,4 keV.

Le nombre de pics détecté pour les échantillons de référence en fonction de la teneur T en fer dissout et en fer particulaire a été préalablement stocké, sous la forme d'un ensemble de données D3, au sein de la mémoire 504M. Les informations de l'ensemble de données D3 sont fournies à un calculateur du détecteur 504 lors de l'étape 900, ce qui permet au calculateur de repérer à quelle teneur de fer correspond le pic détecté pour le niveau d'énergie de 6,4 keV. Cette valeur de la teneur en fer T peut alors être intégrée au signal S50.

En pratique, la méthode automatisée de suivi en ligne de l'évolution de la teneur en fer d'un corps liquide comprend deux parties principales, à savoir la partie A, représentée à la figure 6, qui correspond au procédé de calibration du capteur et la partie B, représentée à la figure 7, qui correspond à la mesure effective de la teneur en fer d'un échantillon, une fois que le capteur 50 a été préalablement calibré. La partie B ne peut être effectuée de façon précise qu'une fois que la partie A a été mise en oeuvre.

Le fait que la méthode de suivi repose sur une détection par fluorescence X au sein du capteur 50 implique qu'il s'agit d'une méthode non destructive, de sorte que l'huile analysée peut être réinjectée vers la machine M.

Les parties A et B de la méthode peuvent être implémentées de façon automatique. En particulier, le procédé de calibration peut être mis en oeuvre automatiquement, avant chaque mesure ou série de mesures de la teneur en fer d'un lubrifiant au sein de l'installation 2. En variante, ce procédé peut être mis en oeuvre à intervalles réguliers, par exemple une fois par jour, toujours automatiquement.

Pour réaliser les étapes 300 à 900, le volume V508 du boîtier 508 peut être alimenté successivement en air et en lubrifiant à analyser, à partir du réservoir 26, moyennant un pilotage adapté des vannes 32, 36, 40 et 44.

L'intensité et la position des pics au sein du spectre S dépend de la matrice de matière environnant les atomes de fer au sein de l'échantillon présent dans le volume V508 lors de l'étape 700. En d'autres termes, des interactions ont lieu entre les atomes de fer présents dans l'échantillon et leur environnement. Ce phénomène est connu sous la dénomination « effet de matrice ». En particulier, les autres atomes présents dans le lubrifiant absorbent des électrons émis par les atomes de fer, ce qui a pour effet d'atténuer le rayonnement X secondaire. D'autre part, les atomes qui absorbent les électrons réémettent eux-mêmes des électrons susceptibles d'exciter les atomes de fer, ce qui participe à une amplification du rayonnement X secondaire. En outre, les atomes de lubrifiant qui entourent les atomes de fer ont également tendance à absorber le rayonnement de fluorescence primaire issu de la source 502.

Lors de l'étape 800 identifiée ci-dessus, il est tenu compte de cet effet de matrice en corrigeant la hauteur du pic correspondant à une énergie de 6,4 keV environ. Cette correction est effectuée sur la base d'un modèle MP préalablement stocké dans la mémoire 504M sous la forme d'un ensemble de données D4. Ce modèle peut être un modèle semi-empirique utilisant plusieurs échantillons de référence, dont les concentrations sont similaires aux échantillons inconnus à analyser, tels qu'envisagés par N. Broll dans « Fluorescence X : de la découverte des rayons de Rontgen aux identités de Tertian » J. Phys. IV, Vol. 06, No.C4, pp C4-583-C4-596, 1996. Le modèle MP peut également être un modèle théorique basé sur l'équation des paramètres fondamentaux des échantillons utilisés, tel qu'envisagé par B. Beckhoff, Habil. B. Kanngiesser, N. Langhoff, R. Weddell et H Wolff dans « Handbook of practical X-ray fluorescence analysis » Berlin, Heidelberg Springer, 2006.

D'autre part, la méthode de suivi en ligne de l'évolution de la teneur en fer de l'huile peut également tenir compte du fait que les lubrifiants d'équipements tels que des éoliennes les plus couramment utilisés sont assez fortement chargés en calcium, ce qui a pour effet de perturber la détection du rayonnement X secondaire issu du fer. Il est donc possible d'établir un modèle NP qui corrèle un spectre établi pour une teneur en fer donnée en l'absence de calcium et un spectre établi dans les mêmes conditions en présence de calcium, dans une ou plusieurs concentration(s) habituellement rencontrée(s) dans un lubrifiant d'équipement industriel. En particulier, le calcium correspond à un niveau d'énergie dans le spectre égal à environ 3 keV. Le modèle NP est stocké dans la mémoire 504M sous la forme d'un ensemble de données D5 et peut être utilisé lors de l'étape 800 ou lors de l'étape 900 pour corriger soit le spectre S soit le calcul de la teneur en fer. Le modèle NP peut être établi de façon théorique ou expérimentale.

En variante, les ensembles de données D4 et D5 sont stockés dans une mémoire de l'unité de commande électronique 22.

L'installation 2 comprend également un premier capteur de niveau 54 et un deuxième capteur de niveau 56 qui permettent respectivement de détecter lorsque la quantité de l'huile dans le réservoir 26 atteint un premier niveau N1 ou un deuxième niveau N2. Les signaux électriques de sortie S54 et S56 des capteurs 54 et 56 sont délivrés à l'unité 22.

En variante, les capteurs 54 et 56 peuvent être remplacés par un unique capteur, tel qu'un capteur de pression, qui permet de détecter lorsque l'huile atteint chacun des deux niveaux N1 et N2 dans le réservoir 26.

Les figures 8 à 11 illustrent schématiquement les étapes successives d'un procédé automatisé mis en oeuvre grâce à l'installation 2 de la figure 1. Ce procédé est automatisé en ce sens qu'il est peut être implémenté, partiellement ou de préférence totalement, sans intervention humaine, sous le contrôle de l'unité 22. Il en va de même pour le procédé explicité ci-après au sujet du deuxième mode de réalisation de l'invention.

Par défaut, et en dehors des phases de prélèvement, l'huile sortant de l'équipement M s'écoule dans la conduite 4, dans le sens de la flèche F1 à la figure 1, de l'équipement M vers le bac de récupération 6, sans être retenue par la vanne 20 qui est en configuration ouverte ou passante, alors que les autres vannes sont fermées.

Lorsqu'il convient de déterminer la teneur en fer de l'huile sortant de l'équipement M, l'unité 22 pilote la vanne 20 à la fermeture, de sorte qu'il est créé dans la conduite 4 une retenue où s'accumule une quantité d'huile, c'est-à-dire de lubrifiant, comme représenté par la partie grisée L à la figure 2.

Dans la configuration de la figure 8, la conduite 4 sert de colonne de décantation et des impuretés 1 s'accumulent au voisinage de la vanne 20, à l'intérieur de la conduite 4 et en partie basse de la quantité de lubrifiant L.

Dans cette première étape représentée par la configuration de la figure 8, les vannes 32 et 40 sont ouvertes, alors que les vannes 36 et 44 sont fermées.

Lorsque le niveau de lubrifiant L dans la conduite ou colonne 4 atteint l'embouchure 282, de l'huile commence à s'écouler à travers la première ligne de dérivation 28, plus particulièrement à travers le filtre 30 et la vanne 32, jusque dans le volume intérieur V26 du réservoir 26 dans lequel l'huile s'écoule par gravité. En effet, le débouché 284 de la première ligne 28 est situé en partie haute du réservoir 26 et l'huile peut s'écouler le long de la paroi du réservoir 26. Comme la vanne 44 est fermée, l'huile remplit progressivement la partie de la deuxième ligne d'évacuation 42 située en amont de la vanne 44, y compris les volumes internes des capteurs 46 et 48, puis le volume intérieur V26, en chassant l'air vers l'atmosphère, à travers la vanne 40. Cette étape correspond à la configuration représentée à la figure 9.

Lorsque le capteur 56 détecte que le niveau N2 d'huile à l'intérieur du réservoir 26 est atteint, l'unité 22 bascule l'installation 2 vers une nouvelle configuration représentée à la figure 10, dans laquelle la vanne 20 passe en configuration ouverte, ce qui permet de vidanger la colonne de décantation en dirigeant le reliquat de la quantité L de lubrifiant présente en amont de la vanne 20 ainsi que les impuretés I vers le bac de récupération 6. L'écoulement dans le sens de la flèche F1 se poursuit donc jusque dans le bac 6. Par ailleurs, les vannes 32 et 40 sont fermées et la vanne 36 est ouverte, ce qui permet de mettre la partie du volume V26 qui n'est pas occupée par le lubrifiant, c'est-à-dire la partie de ce volume V26 située au-dessus du niveau N2, sous une pression d'air P1 égale à celle de la source d'air 12, qui, dans l'exemple, vaut 7 bars absolus.

Ceci étant fait, l'unité 22 fait passer l'installation 2 à une étape suivante, représentée par la configuration de la figure 11, où la vanne 44 est ouverte, les autres vannes conservant leur état de la configuration de la figure 4. Dans ce cas, la pression P1 de l'air dans la partie supérieure du volume V26 a pour effet de pousser l'huile dans la deuxième ligne d'évacuation 42, à travers les capteurs 46, 48 et 50, ce qui permet à ces capteurs de fournir à l'unité 22 des signaux S46, respectivement S48 et S50, représentatifs des paramètres qu'ils ont détectés.

Le cas échéant, les signaux S46, S48 et S50 peuvent être traités dans l'unité 22 pour déterminer les valeurs des paramètres contrôlés, notamment par comparaison avec des valeurs connues pour des lubrifiants de référence.

Les signaux S46, S48 et S50, ou des signaux extrapolés à partir de ces signaux, peuvent être fournis à l'extérieur de l'installation 2 sous la forme d'un signal conjugué S2, exploitable par une unité centrale de contrôle de l'équipement M.

En pratique, la section de passage du capteur 50 de teneur en fer est d'environ 70 mm². Elle peut aller jusqu'à 200 mm². Dans tous les cas, il convient de pouvoir alimenter cette section de passage avec un débit suffisant, pendant une durée suffisante à la réalisation de la mesure de la teneur globale en fer. En variante, il en va de même pour le capteur 48 d'indice de basicité. La construction de l'installation avec le réservoir 26 permet de créer une réserve formant un « tampon » d'huile, sous la forme de la quantité d'huile L1 contenue dans le réservoir 26 dans la configuration de la figure 4. Une partie de cette réserve d'huile L1 peut être déversée, de façon continue ou séquentielle, dans la deuxième ligne d'évacuation 42 pour que les capteurs 48 et 50 disposent d'une quantité suffisante d'huile à analyser.

A partir de la configuration de la figure 11, il est possible, dans une étape subséquente, de poursuivre la vidange du réservoir 26 et de l'intégralité de la deuxième ligne d'évacuation 42 en maintenant la vanne 44 ouverte et en poursuivant l'injection d'air comprimé à travers la vanne 36.

En variante, il est possible d'arrêter la vidange du réservoir 26 lorsque le niveau d'huile atteint le niveau N1, de façon à conserver en permanence une quantité L2 d'huile dans la deuxième ligne d'évacuation 42, en particulier dans les capteurs 46, 48 et 50 dont les parties actives au contact de l'huile ne risquent pas de sécher. Ceci évite notamment le dépôt de traces d'huile sur la paroi 510 du capteur 50. Si cette deuxième approche est sélectionnée, une certaine quantité d'huile doit être utilisée lors d'une prochaine mesure, pour nettoyer préalablement la deuxième ligne d'évacuation 42 et ne pas perturber la prochaine mesure.

Selon une variante non représentée de l'invention, l'installation 2 peut être modifiée pour que les première et deuxième lignes 28 et 42 se rejoignent au niveau d'un embranchement en T disposé en partie basse du réservoir 26. Dans ce cas, le réservoir 26 est rempli par le bas. En outre, les capteurs de niveau 54 et 56 peuvent être remplacés par un capteur de la pression interne au réservoir 26.

Selon une autre variante non représentée de l'invention, plusieurs conduites comparables à la conduite 4 sont utilisées, chacune d'entre elles étant prévue pour collecter de l'huile à partir d'une partie de l'équipement M. Dans ce cas, chacune de ces conduites est équipée d'une vanne comparable à la vanne 20, ce qui leur permet d'alimenter, en parallèle, un réservoir tampon commun comparable au réservoir 26. Comme dans le premier mode de réalisation, un capteur, identique au capteur 50, est disposé dans une conduite d'évacuation du lubrifiant à partir de ce réservoir tampon.

L'invention est décrite ci-dessus dans le cas où le capteur 50 sert à déterminer la teneur en fer d'un lubrifiant. Elle est toutefois applicable pour la détermination de la teneur d'un lubrifiant en un autre élément chimique prédéterminé, par exemple en calcium, soufre, vanadium, chrome, molybdène, cuivre, argent, étain, aluminium, nickel, zinc, plomb ou en phosphore. Dans tous les cas, le matériau constitutif du boîtier est adapté à l'élément chimique en question, de même que le ou les niveaux d'énergie utilisés à l'étape 800. Par « adapté à l'élément chimique », on entend que le matériau constitutif du boîtier ne comprend pas cet élément chimique, ce qui évite une perturbation de la mesure.

L'invention est décrite ci-dessus dans le cas de son utilisation pour un équipement M de type éolienne. Elle est toutefois applicable à d'autres équipements, par exemple un dispositif auxiliaire ou accessoire de navire, ainsi qu'une boîte à vitesses, notamment une boîte à vitesse d'hydrolienne ou d'éolienne, ou dans une raffinerie.

Dans ce qui précède, les mots « huile » et « lubrifiant » sont utilisés indistinctement car, au sens de l'invention, une huile est un lubrifiant. L'invention est toutefois applicable à d'autres corps liquides, tels que du fuel lourd ou léger, avant ou après raffinage pour contrôler le taux d'éléments soufrés. L'invention est applicable aux huiles pour transmissions, aux huiles pour engrenages, aux huiles pour compresseurs, aux huiles hydrauliques, aux huiles de turbine ou encore aux huiles pour centrifugeuse. L'invention est également applicable au suivi en continu de la pollution de l'eau par des éléments métalliques comme le plomb et, plus généralement, à tout corps liquide.

Dans ce qui précède, les expressions « teneur en fer », « teneur globale en fer » et « teneur en fer dissout et/ou particulaire » sont utilisées indistinctement, de même que les expressions « teneur en élément chimique prédéterminé » et « teneur globale en élément chimique prédéterminé ».

Le procédé de calibration de l'invention peut être mis en oeuvre pour un capteur 50 destiné à être utilisé dans un environnement autre que l'installation 2. Dans ce cas, lors de l'étape 300 d'établissement du deuxième spectre S2 de rayonnement secondaire, il est possible que le volume interne V508 du boîtier 508 soit rempli d'un gaz autre que de l'air.

Le capteur 50 est destiné, notamment, à la mise en oeuvre du procédé de calibration et de la méthode de suivi mentionnés ci-dessus. Il peut également être utilisé dans un autre contexte.

Les caractéristiques des modes de réalisation et variantes envisagées ci-dessus peuvent être combinées pour générer de nouveaux modes de réalisation de l'invention.

## Revendications

1. Procédé de calibration d'un capteur de détermination de la teneur en un élément chimique prédéterminé d'un corps liquide, ce capteur utilisant la technologie de fluorescence X et comprenant une source de rayons X (502), un détecteur de rayons X (504) et une cellule (506) destinée à contenir un échantillon de corps liquide à analyser, cette cellule incluant un boîtier (508) définissant un volume interne (V508) de réception de l'échantillon, caractérisé en que le procédé comprend au moins les étapes suivantes consistant à :
a) établir (100), en intégrant des mesures effectuées par le détecteur de rayons X (504) sur une période prédéterminée pour chaque niveau d'énergie (E) qui correspond à une longueur d'onde (λ) caractéristique d'un composant chimique, un premier spectre (S1) de rayonnement X secondaire comprenant des niveaux d'énergie (E₁, E₂, E₃, E₄) correspondant aux composants du matériau du boîtier (508) ;
b) stocker (200) dans une mémoire le premier spectre (S1), sous la forme d'un ensemble de données (D1) ;
c) faire fonctionner (300) le capteur (50), alors que le volume interne (V508) du boîtier ne contient pas de d'échantillon du corps liquide et établir un deuxième spectre (S2) de rayonnement X secondaire
d) modifier (400) le deuxième spectre (S2) en utilisant le premier spectre (S1) stocké à l'étape b) comme spectre de base, pour aligner les niveaux d'énergie remarquables (E'₁, E'₂, E'₃, E'₄) du deuxième spectre sur ceux (E₁, E₂, E₃, E₄) du premier spectre, en repérant quel pic du premier spectre (S1) correspond à chaque pic du deuxième spectre (S2) et en corrigeant les valeurs des niveaux d'énergie remarquables (E'₁, E'₂, E'₃, E'₄) du deuxième spectre pour les disposer, sur l'axe des abscisses E(λ), au même emplacement que les niveaux d'énergie remarquables (E₁, E₂, E₃, E₄) du premier spectre ;
e) enregistrer (500) le deuxième spectre modifié (S2') comme spectre de référence pour le capteur.

2. Procédé selon la revendication 1, **caractérisé en ce que**, lors de l'étape b) (300), le volume interne (V508) du boîtier (508) contient exclusivement un gaz ou un mélange gazeux, inerte aux rayons X, notamment de l'air.

3. Procédé selon l'une des revendications précédentes, **caractérisé en ce que** le boîtier (508) est métallique et les niveaux d'énergie remarquables (E₁, E₂, E₃, E₄) du premier spectre (S1) comprennent des niveaux d'énergie de métaux composant le boîtier.

4. Procédé selon la revendication 3, **caractérisé en ce que** l'élément chimique prédéterminé est le fer et **en ce que** les métaux dont les niveaux d'énergie (E₁, E₂, E₃, E₄) constituent le premier spectre (S1) ne comprennent pas le fer.

5. Procédé selon l'une des revendications précédentes, **caractérisé en ce que** le premier spectre (S1) comprend également au moins un niveau d'énergie (E₁₀, E₁₁) correspondant au matériau d'une anode de la source de rayons X (502).

6. Méthode automatisée de suivi en ligne de l'évolution de la teneur (T) en un élément chimique prédéterminé d'un corps liquide circulant dans un équipement (M) au moyen d'une installation comprenant un capteur (50) de la teneur en élément chimique prédéterminé de ce corps liquide, ce capteur utilisant la technologie de fluorescence X et comprenant une source de rayons X (502), un détecteur de rayons X (504) et une cellule (506) destinée à contenir un échantillon de liquide à analyser et incluant un boîtier définissant un volume interne (V508) de réception de l'échantillon, **caractérisée en ce que** la méthode comprend la mise en oeuvre préalable (A) d'un procédé de calibration du capteur (50) selon l'une des revendications précédentes, ainsi qu'au moins une série (B) d'étapes (600-900) consistant à déterminer, grâce au capteur préalablement calibré, la teneur (T) en élément chimique prédéterminé d'un échantillon de corps liquide présent ou circulant dans le volume interne (V508) du boîtier (508), cette série (B) d'étapes (600-900) comprenant au moins les étapes suivantes consistant à
f) démarrer (600) le capteur (50) ;
g) acquérir (700) un spectre (S) avec un fonctionnement analogue à celui mis en oeuvre à l'étape c) ;
h) comparer (800) le spectre acquis à l'étape g) au spectre de référence (S2') enregistré à l'étape e) ; et
i) comparer (900) un nombre de coups, correspondant à un niveau d'énergie représentatif de l'élément chimique prédéterminé, au nombre de pics détectés pour des échantillons de référence.

7. Méthode selon la revendication 6, **caractérisée en ce que** l'élément chimique prédéterminé est le fer ou un autre élément chimique et **en ce que** la série (B) d'étapes (600-900) comprend la détection (800) de pics pour un niveau d'énergie égal à environ 6,4 keV et/ou 7,06 keV dans un spectre (S) de rayonnement X secondaire émis par le corps liquide pour le fer, ou aux raies spécifiques pour l'autre élément chimique prédéterminé.

8. Méthode selon l'une des revendications 6 ou 7, **caractérisée en ce que** lors de la série (B) d'étapes (600-900), il est tenu compte d'un éventuel effet de matrice au sein de l'échantillon en appliquant une correction sur les niveaux d'énergie détectés par le capteur (50), sur la base d'un modèle prédéterminé (MP) d'interaction entre les composants de l'échantillon et/ou les composants du boîtier (508).

9. Méthode selon la revendication 8, **caractérisée en ce que** lors de la série (B) d'étapes (600-900), il est tenu compte de la concentration en calcium de l'échantillon en appliquant une correction sur les niveaux d'énergie détectés par le capteur (50), sur la base d'un modèle prédéterminé (NP).

10. Méthode selon l'une des revendications 6 à 9, **caractérisée en ce que** la détermination de la teneur (T) en élément chimique prédéterminée de l'échantillon est effectuée de façon dynamique ou statique.

## Patentansprüche

1. Verfahren zur Kalibrierung eines Sensors zur Bestimmung des Gehalts eines flüssigen Körpers an einem vorbestimmten chemischen Element, wobei dieser Sensor die Röntgenfluoreszenztechnologie verwendet und eine Röntgenstrahlenquelle (502), einen Röntgenstrahlendetektor (504) und eine Zelle (506) umfasst, die dazu bestimmt ist, eine Probe des zu analysierenden flüssigen Körpers zu enthalten, wobei diese Zelle ein Gehäuse (508) einschließt, das ein Innenvolumen (V508) zur Aufnahme der Probe definiert, **dadurch gekennzeichnet, dass** das Verfahren mindestens die folgenden Schritte umfasst:
a) Erstellen (100), durch Integrieren von Messungen, die von dem Röntgenstrahlendetektor (504) über einen vorbestimmten Zeitraum für jedes Energieniveau (E) durchgeführt werden, das einer Wellenlänge (λ) entspricht, die für eine chemische Komponente charakteristisch ist, eines ersten Spektrums (S1) sekundärer Röntgenstrahlung mit Energieniveaus (E₁, E₂, E₃, E₄), die den Komponenten des Materials des Gehäuses (508) entsprechen;
b) Speichern (200) des ersten Spektrums (S1) in einem Speicher in Form eines Datensatzes (D1);
c) Betreiben (300) des Sensors (50), während das Innenvolumen (V508) des Gehäuses keine Probe des flüssigen Körpers enthält und Erstellen eines zweiten Spektrums (S2) der sekundären Röntgenstrahlung;
d) Modifizieren (400) des zweiten Spektrums (S2) unter Verwendung des in Schritt b) gespeicherten ersten Spektrums (S1) als Basisspektrum, um die bemerkenswerten Energieniveaus (E'₁, E'₂, E'₃, E'₄) des zweiten Spektrums mit denen (E₁, E₂, E₃, E₄) des ersten Spektrums abzugleichen, durch Ermitteln, welcher Peak des ersten Spektrums (S1) mit jedem Peak des zweiten Spektrums (S2) übereinstimmt und durch Korrigieren der Werte der bemerkenswerten Energieniveaus (E'₁, E'₂, E'₃, E'₄) des zweiten Spektrums, um sie auf der Abszissenachse E(λ) an derselben Stelle wie die bemerkenswerten Energieniveaus (E₁, E₂, E₃, E₄) des ersten Spektrums anzuordnen;
e) Aufzeichnen (500) des zweiten modifizierten Spektrums (S2') als Referenzspektrum für den Sensor.

2. Verfahren nach Anspruch 1, **dadurch gekennzeichnet, dass** in Schritt b) (300) das Innenvolumen (V508) des Gehäuses (508) ausschließlich ein gegenüber Röntgenstrahlen inertes Gas oder Gasgemisch, insbesondere Luft, enthält.

3. Verfahren nach einem der vorhergehenden Ansprüche, **dadurch gekennzeichnet, dass** das Gehäuse (508) metallisch ist und die bemerkenswerten Energieniveaus (E₁, E₂, E₃, E₄) des ersten Spektrums (S1) Energieniveaus von Metallen umfassen, aus denen das Gehäuse besteht.

4. Verfahren nach Anspruch 3, **dadurch gekennzeichnet, dass** das vorbestimmte chemische Element Eisen ist und dass die Metalle, deren Energieniveaus (E₁, E₂, E₃, E₄) das erste Spektrum (S1) bilden, kein Eisen umfassen.

5. Verfahren nach einem der vorhergehenden Ansprüche, **dadurch gekennzeichnet, dass** das erste Spektrum (S1) ebenfalls mindestens ein Energieniveau (E₁₀, E₁₁) umfasst, das dem Material einer Anode der Röntgenstrahlenquelle (502) entspricht.

6. Automatisiertes Verfahren zur Online-Verfolgung der Entwicklung des Gehalts (T) an einem vorbestimmten chemischen Element eines flüssigen Körpers, der in einer Ausrüstung (M) zirkuliert, mittels einer Anlage, die einen Sensor (50) für den Gehalt dieses flüssigen Körpers an dem vorbestimmten chemischen Element umfasst, wobei dieser Sensor die Röntgenfluoreszenztechnologie verwendet und eine Röntgenstrahlenquelle (502), einen Röntgenstrahlendetektor (504) und eine Zelle (506) umfasst, die dazu bestimmt ist, eine zu analysierende Flüssigkeitsprobe zu enthalten und ein Gehäuse einschließt, das ein Innenvolumen (V508) zur Aufnahme der Probe definiert, **dadurch gekennzeichnet, dass** das Verfahren die vorherige Durchführung (A) eines Verfahrens zur Kalibrierung des Sensors (50) nach einem der vorhergehenden Ansprüche sowie mindestens eine Reihe (B) von Schritten (600-900) umfasst, die darin bestehen, dank des zuvor kalibrierten Sensors den Gehalt (T) eines vorbestimmten chemischen Elements in einer Probe eines flüssigen Körpers, der sich im Inneren des Gehäuses befindet oder in dem Innenvolumen (V508) des Gehäuses (508) zirkuliert, zu bestimmen, wobei diese Reihe (B) von Schritten (600-900) mindestens die folgenden Schritte umfasst:
f) Starten (600) des Sensors (50);
g) Erfassen (700) eines Spektrums (S) mit einer ähnlichen Funktionsweise wie in Schritt c);
h) Vergleichen (800) des in Schritt g) erfassten Spektrums mit dem in Schritt e) aufgezeichneten Referenzspektrum (S2'); und
i) Vergleichen (900) einer Anzahl von Treffern, die einem Energieniveau entsprechen, das für das vorbestimmte chemische Element repräsentativ ist, mit der Anzahl von Peaks, die für Referenzproben erfasst wurden.

7. Verfahren nach Anspruch 6, **dadurch gekennzeichnet, dass** das vorbestimmte chemische Element Eisen oder ein anderes chemisches Element ist und dass die Reihe (B) von Schritten (600-900) das Bestimmen (800) von Peaks für ein Energieniveau von etwa gleich 6,4 keV und/oder 7,06 keV in einem Spektrum (S) der sekundären Röntgenstrahlung, die von dem flüssigen Körper für Eisen emittiert wird, oder den spezifischen Linien für das andere vorbestimmte chemische Element umfasst.

8. Verfahren nach einem der Ansprüche 6 oder 7, **dadurch gekennzeichnet, dass** in der Reihe (B) von Schritten (600-900) ein möglicher Matrixeffekt innerhalb der Probe durch Anwenden einer Korrektur auf die vom Sensor (50) erfassten Energieniveaus auf der Basis eines vorbestimmten Modells (MP) der Interaktion zwischen den Komponenten der Probe und/oder den Komponenten des Gehäuses (508) berücksichtigt wird.

9. Verfahren nach Anspruch 8, **dadurch gekennzeichnet, dass** in der Reihe (B) von Schritten (600-900) die Kalziumkonzentration in der Probe durch Anwenden einer Korrektur auf die von dem Sensor (50) erfassten Energieniveaus auf der Basis eines vorbestimmten Modells (NP) berücksichtigt wird.

10. Verfahren nach einem der Ansprüche 6 bis 9, **dadurch gekennzeichnet, dass** die Bestimmung des Gehalts (T) eines vorbestimmten chemischen Elements in der Probe dynamisch oder statisch durchgeführt wird.

## Claims

1. A process for calibrating a sensor for determining the content of a predetermined chemical element in a liquid body, said sensor using X-ray fluorescence technology and comprising an X-ray source (502), an X-ray detector (504) and a cell (506) intended to contain a sample of liquid body to be analyzed, said cell (508) in turn including a casing (508) that defines an internal volume (V508) for receiving the sample, **characterized in that** the process includes at least the following steps consisting in :
a) establishing (100), by incorporating the measurements done by the X-ray detector (504) for each energy level (E) that corresponds to a wavelength (λ) characteristic of a chemical component, a first secondary X-ray spectrum (S1) comprising energy levels (E₁, E₂, E₃, E₄) corresponding to the components of the material of the casing (508);
b) storing (200) in a memory the first spectrum (S1), in the form of a data set (D1);
c) operating (300) the sensor (50), while the internal volume (V508) of the casing does not contain a sample of the liquid body and establishing a second secondary X-ray spectrum (S2);
d) modifying (400) the second spectrum (S2), using the first spectrum (S1) as a base spectrum, for aligning the notable energy levels (E'₁, E'₂, E'₃, E'₄) of the second spectrum on those (E₁, E₂, E₃, E₄) of the first spectrum, by identifying which peak of the first spectrum (S1) corresponds to each peak of the second spectrum (S2) and by correcting the values of the notable energy levels (E'₁, E'₂, E'₃, E'₄) of the second spectrum to arrange them, on the x-axis E(λ), in the same location as the notable energy levels (E₁, E₂, E₃, E₄) of the first spectrum S1;
e) recording (500) the modified second spectrum (S2') as reference spectrum for the sensor.

2. The process according to claim 1, **characterized in that**, during step b) (300), the internal volume (V508) of the casing (508) exclusively contains a gas or a gaseous mixture, inert with respect to the X-rays, in particular air.

3. The process according to one of the preceding claims, **characterized in that** the casing (508) is made from metal and the notable energy levels (E₁, E₂, E₃, E₄) of the first reference spectrum (S1) comprise energy levels of metals making up the box.

4. The process according to claim 3, **characterized in that** the predetermined chemical element whereof one wishes to determine the content is iron and the metals whose energy levels (E₁, E₂, E₃, E₄) make up the first spectrum (S1) do not comprise iron.

5. The process according to one of the preceding claims, **characterized in that** the first spectrum (S1) also comprises at least one energy level (E₁₀, E₁₁) corresponding to the material of an anode of the X-ray source (502).

6. An automated method for online monitoring of the changes in content (T) of a predetermined chemical element of a liquid body circulating in a piece of equipment (M) using an installation comprising a sensor (50) for the content of a predetermined chemical element of said liquid body, said sensor using X-ray fluorescence technology and comprising an X-ray source (502), an X-ray detector (504) and a cell (506) intended to contain a sample of liquid body to be analyzed and including a casing defining an internal volume (V508) for receiving said sample, **characterized in that** the method comprises the prior implementation (A) of a process for calibrating the sensor (50) according to one of the preceding claims, as well as at least one series (B) of steps (600-900) consisting of determining, owing to the sensor previously calibrated, the content (T) in predetermined chemical element of a sample of liquid body present or circulating in the internal volume (V508) of the casing (508), this series (B) of steps (600-900) including ate least the following steps consisting in
f) starting (600) the sensor (50);
g) acquiring (700) a spectrum (S) with an operation similar to that implemented at step c);
h) comparing (800) the spectrum acquired at step g) with the reference spectrum (S'2) recorded at step e); and
i) comparing (900) a number of blows, corresponding to an energy level representative of the predetermined chemical element, to the number of peaks detected for reference samples.

7. The method according to claim 6, **characterized in that** the predetermined chemical element is iron, or another chemical element, and the series (B) of steps (600-900) comprises detecting (800) peaks for an energy level equal to about 6.4 keV and/or 7.06 keV in a secondary X-ray radiation spectrum (S) emitted by the liquid body for iron, or specific rays for the other predetermined chemical element.

8. The method according to one of claims 6 or 7, **characterized in that** during the series (B) of steps (600-900), a potential matrix effect is taken into account within the sample by applying a correction on the energy levels detected by the sensor (50), based on a predetermined interaction model (MP) between the components of the sample and/or the components of the casing (508).

9. The method according to claim 8, **characterized in that** during the series (B) of correction steps (600-900), the calcium concentration of the sample is further taken into account by applying a correction on the energy levels detected by the sensor (50), based on a predetermined model (NP).

10. The method according to one of claims 6 to 9, **characterized in that** the determination of the content (T) in predetermined chemical element is made dynamically or statically.
